# EUROPEAN PATENT SPECIFICATION

(11) **EP 0 944 834 B1**
(45) Date of publication and mention of the grant of the patent: **23.10.2002**
(21) Application number: 97945953.4
(22) Date of filing: 27.11.1997
(51) Int. Cl.: G01N 33/543, G01N 33/53

(54) **SURFACE PATTERNING OF AFFINITY REAGENTS USING PHOTOABLATION**
OBERFLÄCHENMUSTER VON AFFINITÄTREAGENTIEN MITTELS PHOTOABLATION
STRUCTURATION DE SURFACE DE REACTIFS D'AFFINITE PAR PHOTOABLATION

(30) Priority: 27.11.1996 GB 9624686
(43) Date of publication of application: 29.09.1999
(73) Proprietor: ECOLE POLYTECHNIQUE FEDERALE DE LAUSANNE (Laboratorie D'Electrochimie), 1015 Lausanne (CH)
(72) Inventor: ROBERTS, Matthew, A., CH-1030 Bussigny (CH); LAEDERACH, Alain, CH-1022 Chavannes (CH); BERCIER, Paul, CH-1018 Lausanne (CH); GIRAULT, Hubert, Hugues, CH-1088 Ropraz (CH); SEDDON, Brian, CH-1414 Ruyres (CH)
(74) Representative: Hanson, William Bennett
(86) International application number: GB9703246
(87) International publication number: WO98023957

(56) References cited:
- WO-A-91/08474
- WO-A-94/08236
- WO-A-94/23295
- WO-A-96/39937
- WO-A-97/22875
- Journal of Electroanalytical Chemistry 2000, 492, 15 - 22

## Description

### Background to the Invention

Many recent advances in chemical analysis have involved the incorporation of biomolecules capable of selective and high affinity binding to analytes of interest. Such devices are often termed biosensors, which involve real-time transduction of the binding event into an electronic signal, but also include analytical technology such as, for example, affinity chromatography, immunoassay and enzyme reaction, as well as nucleic acid hybridization, which can be performed in analytical polymerase chain reaction (PCR) devices. Bioanalytical devices utilizing this technology have been applied to a wide range of applications in medicine, agriculture, industrial hygiene, and environmental protection. There is also growing interest in the use of modified materials in bio-medical implants, prosthetic devices, catheters, etc. Furthermore, there is an emerging field of bio-electronic and bio-mimetic materials, which can be used for information processing, that rely on the transduction of some molecular recognition event¹.

Recently, there has also been intense activity in the miniaturization of chemical instrumentation. Efforts have been made to reduce whole laboratory systems on to microchip substrates often utilizing capillary electrophoresis (CE) as the principal analytical technique². These systems have been termed micro-Total Analytical Systems (µ-TAS)³. Some promising bioanalytical applications have already been demonstrated which are based on immobilized receptors within microfabricated fluid handling platforms⁴⁻⁷.

Immobilization technology is critical to the further development of all of these technologies. In many cases, commercialization of new ideas has been hampered by either inadequate, unstable, or unreproducible immobilization of the biomolecule components⁸. Immobilization has been accomplished in the past by adsorption, entrapment, non-covalent binding, and covalent conjugation but usually only by application of bulk solution techniques and without the ability for careful spatial delineation of patterns⁹.

Therefore, there exists great interest in developing techniques for the selective and site-specific immobilization of biomolecule reagents on component surfaces of analytical devices. A very recent advance in patterned immobilization uses bacterial cells to mask chemical modifications to polymer surfaces¹⁰. After the bacteria are removed, the reactive polymer will bind fluorescently labelled antibodies within the bacteria-sized regions on polymer surfaces. This treatment still depends on multistep wet chemical treatments and is essentially a detection technique for bacteria. With reference to patterned nucleic acid immobilization, a recent review of promising new DNA sequencing technology has called for the development of microfabricated arrays of immobilized nucleic acid sequences so that parallel and automated sequencing apparatus can be created¹¹. Therefore, the current state of the art can be seen as actively searching for new patterned immobilization technology.

Ligler, F. S. et al, U.S. Patent No. 5,391,463 describes a patterning technique for covalently attaching biomolecules onto silicon dioxide surfaces which have been covalently modified with heterobifunctional cross-linking reagents. The UV-irradiation of such surfaces, containing thiol, epoxy, or vicinal diol functionalities, was shown to be capable of patterning biomolecules with the use of a photomask. However, this method suffers from its dependence on the covalent modification of substrate material before irradiation and from the covalent chemistry required to attach biomolecules after the irradiation step. Furthermore, although pattern areas which were exposed to UV-irradiation became more resistant to non-specific protein adsorption this effect was still significant and measurable (between 13 and 54% of non-irradiated areas depending on the cross-linking molecule used). It is also important to note that the disclosed technique of Ligler et al is clearly not UV-photoablation but only UV-irradiation. UV-photoablation requires significantly higher laser pulse energies then were used in that study and is a completely distinct physical phenomenon, which the present inventors use to advantage in the bio-patterning of biomolecules and chemical substances.

WO 94/23295 describes a substrate with a photoablated hole. Antibodies may be attached to the inside surface of the hole or in the vicinity of the hole, but not to a surface which has been made rugged by photoablation.

WO 94/08236, WO 91/08474 and WO 96/39937 published on 19^{th} December 1996 all describe substrates which have been photoablated and to which a reagent is attached, but not to the photoablated region of the substrate.

WO 97/22875 published on 26^{th} June 1997 describes a substrate which has been laser hydrophilised and to which a reagent is attached.

### Summary of the Invention

Accordingly, it is one object of the invention to provide a novel method for the patterning of biomolecules and chemical substances in 2 and 3-dimensional patterns on UV-absorbing substrate materials.

It is another object of the present invention to provide material surfaces with enhanced immobilization capacity for biomolecules such as proteins, nucleic acids, and lipid films by physical adsorption.

It is another object of the present invention to provide material surfaces with enhanced functionalization of chemical groups for subsequent covalent conjugation procedures.

It is another object of the invention to provide surfaces with protective layers such as adsorbed non-specific protein layers, chemically conjugated lipid films, laminated polymers, adhesives, or photoresists which can be patterned using UV-laser photoablation.

It is yet another object of the invention to utilize the UV-laser ablated, protected surfaces as a contact mask for the subsequent patterning of biomolecules and chemical substances onto the underlying polymer substrate.

These and other objects, which will become apparent during the following detailed description of the present invention, have been achieved by the inventors' use of UV-laser photoablation (as opposed to simple irradiation) enabling enhanced functionalization of polymer surfaces, enhanced biomolecule immobilization capacity, and patterning, with µm (micron) resolution, using attached protective layers.

The present invention provides a method for patterning an affinity reagent as set forth in claim 1, and a biological or chemical sensor as set forth in claim 14.

The present invention utilizes a UV-excimer laser induced photoablative process to produce contact masks through protective layers directly over UV-absorbing substrate materials and simultaneously induces such substrates to undergo physical and chemical changes which show large increases in immobilization capacity of biomolecules. The present invention utilizes UV-laser fluences greater than 0.5J cm⁻² per pulse which is clearly in the photoablative regime and not simply UV-irradiation. The present invention does not need to rely solely on covalent attachment of biomolecules but can also utilize physical adsorption or non-covalent binding for simple single process immobilization techniques. The present invention offers ease-of-use while maintaining a UV-laser's ability to photoablatively define materials into reactive and non-reactive areas with µm (micron) or 1 µm (sub-micron) resolution.

### Brief Description of the Drawings

The invention will now be described by way of example only with reference to the accompanying drawings, in which:-
Figures 1a and 1b are schematic perspective views of a UV Laser micromachining process according to an embodiment of the invention;
Figure 2a to 2c show UV-laser bio-patterning of protein-blocked polymer surfaces;
Figure 3a to 3c show UV-laser bio-patterning of laminate-blocked polymer surfaces;
Figure 4a is a light micrograph showing avidin bio-patterning using UV-laser photoablation;
Figure 4b shows a control in which no avidin was used;
Figure 5 is a voltammogram demonstrating β-Galactosidase bio-patterning using UV-laser photoablation;
Figures 6a to 6c are schematic diagrams of a UV-laser defined microchannel for concentration and separation of analytes in solution;
Figure 7 is a scanning electron micrograph (SEM) of a UV-laser fabricated micro-well array for nucleic acid analysis; and
Figure 8 shows a sandwich immunoassay facilitated by UV-laser patterning.

### Detailed Description of the Preferred Embodiments

### Machining and characterization of UV-laser photoablated micro-patterns.

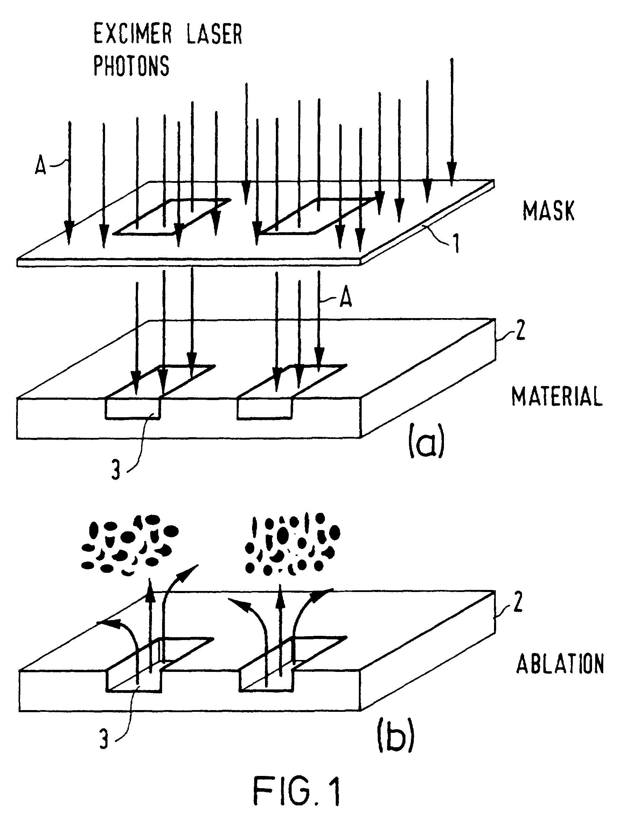

Structure may be formed in various commercially available polymers by photoablation upon interaction with UV laser radiation (λ < 330nm; fluence > 0.5 J cm⁻² per pulse). These have been reviewed¹⁴, but include cellulose acetate, nitrocellulose, polycarbonate, polyimide, poly(methyl methacrylate) (PMMA), polystyrene, poly(ethylene terephthalate) (PET), and poly(tetrafluoroethylene). The photoablation process involves absorption in the UV region with concomitant electronic transitions from the ground singlet state to the first excited singlet states, rapid bond breaking within the long chain polymer molecules, and then ejection of substrate, in a mini-explosion, from the surface leaving a photoablated cavity¹⁴ 3, as depicted in Figure 1b.

The laser energy can be specifically patterned using a photomask with the subsequent generation of microcavities and channels of various geometry. The laser energy may also be patterned in even smaller dimensions by using fiber-optic light guides. Such light guides are capable of handling UV-laser energy of several hundred mJ per pulse and can be fashioned with small tips, using techniques known to those skilled in the art, to have an aperture well within the sub-micron regime (250 - 1000 nm). By positioning such light guides tips very close to the surface of the UV-absorbing substrate, it is possible to accurately and reproducibly fabricate structures with 2-dimensional resolution very close to the diameter of the light guide aperture.

The resulting structures are generally characterized as having little thermal damage, straight vertical walls, and well defined depth. Patterns can be defined well into the sub-micron region, however, a general working range for the patterning of biological or chemical substances will be given here as 1 - 1000 µm.

For the experiments described here, it is necessary only to use simple, line and hole, mask geometry, however, those skilled in the art will appreciate that more complicated masks can be easily fabricated through other microfabrication technologies such as contact lithography. Figures 1a and 1b show (not to scale) linear patterns for which a 10 X 0.4 mm mask¹ was mechanically fabricated in copper foil. For circular patterns, 1, 5, and 10 mm diameter circular masks (not shown) were machine drilled in 5 mm thick steel plates. An excimer laser (LPX 205 i, Lambda Physik GmbH, Goettingen) is used to project UV laser radiation (193 nm, pulse rate = 10 - 50 Hz), shown by the arrows A, through these masks, a 10:1 telescopic objective, and then onto various polymer substrates 2 (Surface energy: 1.6J cm⁻² per pulse). Therefore, each laser pulse will produce an ablated cavity 3 with a cross-sectional area of 1 X 0.04 mm when using the line mask shown in Figure 1a and 0.1, 0.5, or 1 mm diameter hole when using the circular masks.

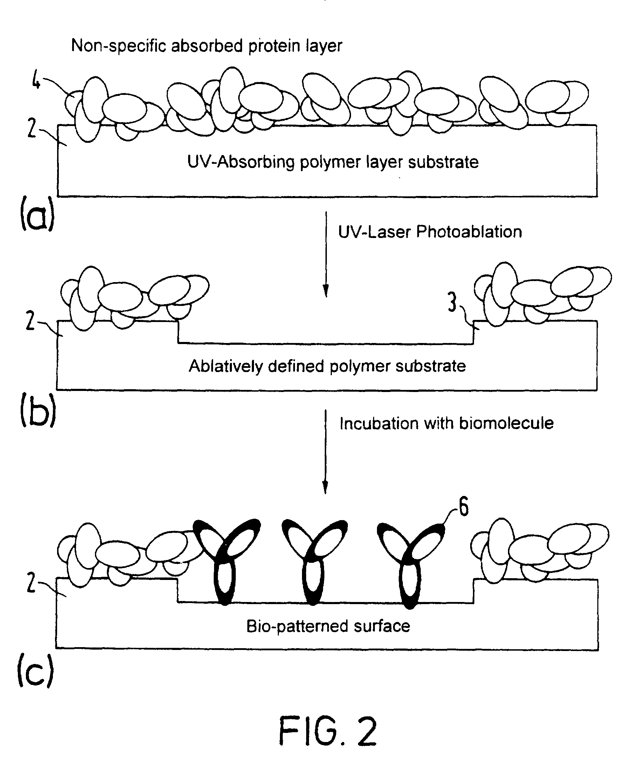

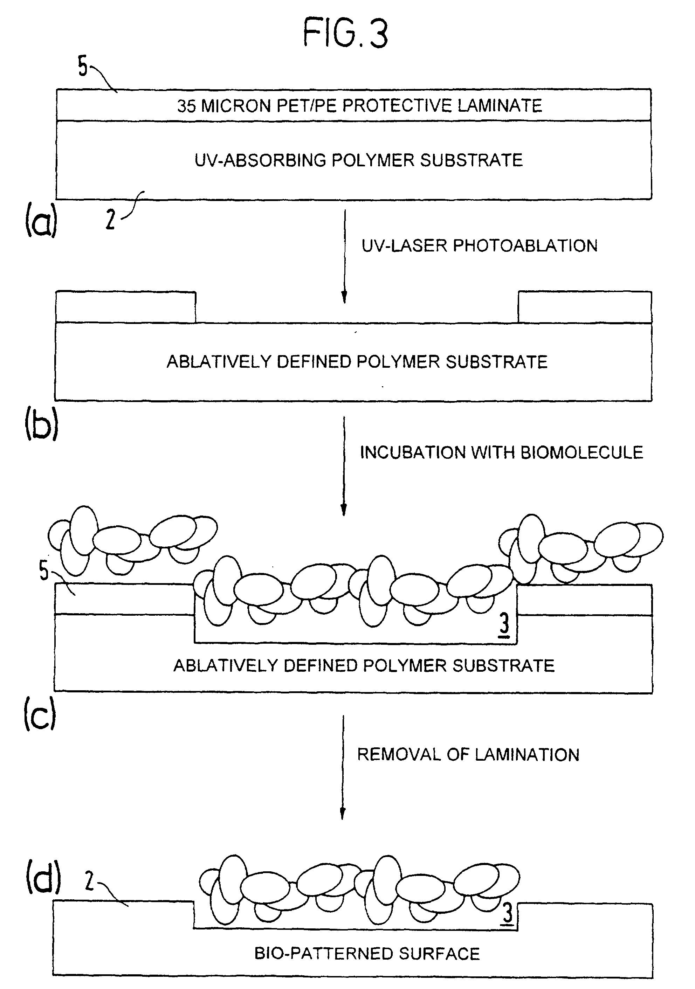

Prior to laser machining, all substrate samples are cleaned by rinsing with distilled water and then drying with pressurized air. Preferably, a protective layer is subsequently applied prior to UV-laser photoablation. This protective layer may be an adsorbed protein layer such as bovine serum albumin, chicken ovalbumin, human serum albumin, any other albumin, non-fat dry milk (NFDM) or any other suitable protein solution capable of efficiently and homogeneously coating the substrate material. An example of an adsorbed protein protective layer 4 is shown in Figure 2a. This protective layer may also be composed of chemically conjugated species such as lipid monolayers, self-assembled layers, fatty acid chains, carbohydrate moieties, or other suitable substances. An alternative protective layer is composed of a polymer laminate such as poly(ethylene terephthalate)/polyethylene (PET/PE) for example, or any other suitable polymer possessing the characteristics of low-temperature and rapid adhesion to the base substrate. An example of a laminated protective layer 5, adherent to a polymer substrate, is shown in Figure 3a. This protective layer may also be composed of other types of materials applied by various techniques which include but are not limited to adhesives, screen printed polymers, and spin-coated photoresist layers. It should be clear to those skilled in the art that the substances mentioned here are a small subset of the possible protective layers that may be applied to UV-laser photoablative substrates, and that these layers should only serve to create a general mask for the substance to be patterned. It should also be realized that such a mask can be both positive or negative, in that the chemical properties of the mask can either block or attract the species of interest in some semi-permanent or complete manner. The subsequent UV-ablative step will serve to define the protected surface into distinct areas possessing this chemical property (non-ablated) and areas that do not (ablated).

To obtain an adsorbed protective layer as shown in Figure 2a, substrate samples can simply be immersed in a high concentration of BSA or NFDM (10 mg/mL in 60 mM phosphate buffered saline (PBS), pH 7.0) at room temperature for 2 - 4 hours. Such solutions of "nonspecific" proteins are known to bind in high concentrations to polymer materials, which in their unablated form have been used for many years as platforms for diagnostic screening^{9,12}. If applied in high concentrations they will adsorb in tightly packed layers seeking to occupy most of the available substrate surface area. Due to the fact that physical adsorption of such substances is semi-permanent, subsequent application of biomolecules will be prevented from close interaction with the substrate material and will only be loosely bound to the protein protective layer. These substances can then be washed from the surface leaving only the protein protected substrate.

To obtain the laminated protective layer shown in Figure 3a, a 35 µm thick layer 4 of PET/PE is rolled onto the substrate² at 125°C for 3 seconds. Either of the techniques of Figures 2 and 3 serve to produce a uniform "positive" mask by blocking any subsequent interaction with receptors, enzymes, or chemical substances of general interest.

For fabrication of patterns using a PET/PE polymer laminate protective layer it is first necessary to ablate this layer completely before patterning the underlying substrate. It was determined that approximately 200 pulses were required to completely ablate the 35 µm thick laminate. Structure such as µ-channel 3 could then be developed in the substrate with additional UV laser pulses. Linear channels were normally fabricated on polycarbonate by pulsing the laser at 50 Hz for three seconds. This corresponds to 150 pulses and was observed to produce linear structures 3 approximately 17 µm deep into the base substrate, after the patterned ablation of the 35 µm-thick laminate. For all linear structures using an adsorbed protein protective layer, only the number of pulses required for the desired structure depth is required, that is to say removal of the protein requires negligible energy. Depending on whether patterning in deep structures or near the surface is desired, this can range from 0 - 200 µm for any of the protective masking techniques.

An affinity reagent was allowed to physically adsorb to the photoablated polymer through laser defined openings in a protective layer. In the case of a PET/PE protective laminate, the lamination is simply peeled off after thorough washing, leaving only laser-defined areas of immobilized affinity reagent as shown in Figure 3d.

It should also be understood by those skilled in the art that more complex geometries can alternatively be produced by utilizing more complicated photomasks. Using contact lithography or other techniques, which are well known in the integrated circuit manufacturing industry, it is possible to create complex ablated patterns. The photomasks are simply placed in front of the excimer laser and repeated pulsing will ablatively pattern the substrate through differnt masks.

### Characterization of Photoablated Cavities

The relationship between the number of laser pulses, laser frequency, and the resulting µ-channel depth has been studied by SEM¹³. Samples were generated using a static substrate and a photomask of the dimensions previously described. High energy UV-laser pulses (2 J cm⁻² per pulse) were focused on a polycarbonate surface and 0 - 1000 pulses were fired at 10, 20, and 50 Hz. Samples were then analyzed by scanning electron microscopy (SEM). The channel depth could be determined in this manner using a high tilt angle (60°) and subsequent measurements of an exposed depth profile. The channel depth was found to be essentially linear (and did not vary with frequency) according to the relationship: Depth (µm) = 0.17*N, where N = the number of laser pulses.

In many cases surface patterning of chemical substances will be the desired result and, therefore, only a low number of pulses will be required to pattern the protective layer, thereby creating little actual structure in the substrate. However, in more complicated devices, especially with fluid handling components, one may use high numbers of pulses differentially applied across the desired 2-dimensional pattern. This embodiment of the present method will allow the patterning of biological and chemical substances at varied depth, thereby, creating 3-dimensional patterning onto the substrate.

It should be noted that the cavity geometries produced by this technique vary little over their entire depth profile, i.e. very straight sidewalls with high aspect ratio are produced, as has been observed by atomic force microscopy (AFM) and SEM¹³. The bottom surface also shows an increased rugosity, and therefore, enhanced surface area, as indicated in Table 1.

**Table 1**

| **Substrate** | Rugosity (µm) | Zeta Potential (ζ in mV) |
|---|---|---|
| Unablated Polymer | 0.01 | NA |
| Polycarbonate (PC) | 0.13 | -52.75 |
| Polystyrene (PY) | 0.13 | -56.22 |
| Cellulose Acetate (CA) | 0.27 | -59.65 |
| Poly(ethylene terephthalate) (PET) | 0.39 | -72.85 |
| BSA coated PC | 0.13 | -30.47 |
| BSA coated PY | 0.13 | -35.71 |
| BSA coated CA | 0.22 | -29.01 |
| BSA coated PET | 0.4 | -39.83 |

The rugosity of unablated polymer substrates was observed as only a 0.01 µm depth variation across a 70 x 70 µm cross-section. Photoablated polycarbonate and polystyrene surfaces appear to have a small undulating rugosity and are very similar to one another with variations of the average depth of 0.13 µm. In contrast, it was observed that cellulose acetate and poly(ethylene terephthalate) surfaces show very pronounced rugged projections on the sub-micron scale and differ in the overall magnitude of this effect. Photoablated cellulose acetate showed an increase in rugosity (0.27 µm total) relative to those just mentioned, however, poly(ethylene terephthalate) showed the largest increase with an overall depth variation of 0.40 µm. From the perspective of developing microfabricated diagnostic assays, the increased surface area seen particularly with photoablated poly(ethylene terephthalate) could be very useful for the deposition of receptor and ligand zones. The magnitude of chemical adsorption will be related to the photoablatively induced charge and the surface area of the resultant microstructure. The increase in surface area due to the observed increase in rugosity leads to increased adsorption.

The adsorptive capacity of photoablated polymers was also studied, using BSA as a model protein intended to show relative differences among the various surfaces studied and the results can be seen in Table 2.

**Table 2**

| Polymer | BSA on Ablated µg/cm² | BSA on Non Ablated µg/cm² | Adsorption factor |
|---|---|---|---|
| Collulose Acetate | 6.7 | 0.5 | 13.2± 7.3 |
| PET | 91.3 | 1.1 | 82.3±15.5 |
| Polystyrene | 145.1 | 2.8 | 52.0± 4.0 |
| Polycarbonate | 174.3 | 4.3 | 40.4± 5.1 |
| Polyimide | 241.1 | 3.7 | 65.1± 9.5 |

Unablated polymer substrates showed little difference in protein adsorption with an average of 1.32 µg BSA / cm² as measured by the Bicinchoninic Acid (BCA) test (Measures reduction of copper by amino acids¹⁴). This value is higher than the 0.4 µg protein / cm² reported in the literature⁹. Our observations were only for BSA, a protein which can be expected to show differences in adsorption when compared to a generalized observation of several different proteins. Although the BCA test measures the protein's ability to reduce copper, which may be enhanced by any denaturation caused by adsorptive immobilization, the test can be expected to accurately determine relative differences in the various surfaces examined here.

The ablated polymers showed dramatic differences in their protein adsorptive properties, ranging from 6.7 - 241.1 µg BSA / cm². This amount is only calculated according to a cross-sectional area on the plane of the initial polymer substrate, however, with photoablative processes, a great change in rugosity is produced and, therefore, the true surface area is much greater. This is one of the principal reasons for the great increase in protein adsorption that we have observed. The order of adsorptive capacity for BSA followed the series; cellulose acetate < PET < polystyrene < polycarbonate < polyimide.

A useful fact to consider when evaluating the process of UV-photoablatively induced changes in immobilization capacity is the ratio of ablated to non-ablated polymer adsorption, as shown in Table 2. This was calculated here by the formula: F = A / N where: A = mass per unit area of protein adsorbed to ablated polymer and N = mass per unit area of protein adsorbed to non ablated polymer. When the adsorption factor (F) is taken into account, the order of the increase in adsorptive capacity is cellulose acetate < polycarbonate < polystyrene < polyimide < PET. All of these polymers have a highly increased rugosity after photoablation as well as increased protein adsorption capacity. The increase is correlated in the sub-series: polycarbonate< polystyrene< PET, however, cellulose acetate is an outlier. Photoablated polymers have also been shown to have a chemically modified surface composition often associated with a surface oxidation when the ablation is carried out in air and therefore the incorporation of atmospheric oxygen during the photoablative process is inferred. The increase in adsorption is likely to be due to not only an increase in rugosity, but also to the specific chemical nature of functional groups generated during UV-laser photoablation.

It should be noted that all of the protein adsorption experiments not only show an increase in adsorptive capacity for photoablated polymers, but also that these regions can be 2-dimensionally patterned by defining the UV-laser beam geometry. All of the BCA tests on photoablated polymers were done over 1 mm diameter ablated wells and after removal of the laminate, no protein could be measured outside of the ablated area. Therefore the 35 µm thick PET/PE laminated protective layer has been observed to completely block non-specific protein binding to areas outside of the laser ablated regions. This is a significant advantage over the prior art in bio-patterning.

It is also demonstrated here that a model receptor, avidin from egg whites, can be immobilized in such microfabricated patterns. This experiment will demonstrate two additional concepts central.to bio-patterning using the technique of UV-laser photoablation; 1) Receptors can be immobilized onto photoablated surfaces while maintaining binding activity for a ligand and 2) that receptors bound in this manner can be patterned with high resolution.

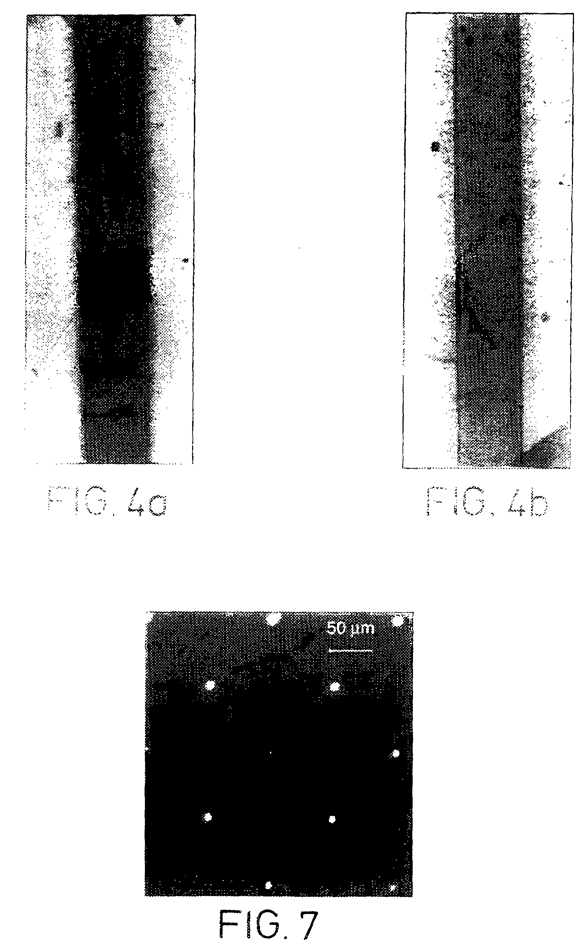

Avidin was allowed to physically adsorb to photoablated polycarbonate through laser defined openings (1 mm diameter microwells) in a PET/PE protective laminate as previously described and shown in Figure 3c. After washing, the lamination was peeled off leaving only laser-defined areas of immobilized avidin as shown in Figure 3d. Biotinylated liposomes have been previously shown to have high affinity for avidin in solution as well as avidin immobilized to polymer surfaces^{12, 15-17}. Ten µL solutions of liposomes, containing 0.1 mol% dipalmitoyl phosphatidyl choline-biotin conjugate (Molecular Probes, Eugene Oregon) and 200 mM encapsulated sulpharhodamine-B, were incubated for 2 hours at room temperature over the photoablated microwells and control surfaces. The entire surface of the polycarbonate sample was then vigorously washed with 67 mM PBS for 60 seconds. Liposomes remained attached to the surface of UV-laser defined microwells through an avidin-biotin non-covalent linkage, as demonstrated in Figure 4a. It was also shown that there was little or no liposome binding to laser-treated areas which were not incubated with avidin, as seen in Figure 4b, showing that in fact the avidin receptor had been immobilized in the laser defined region and was responsible for capturing biotinylated liposomes. Furthermore, it can be seen in Figure 4a, that the borders between ablated and non-ablated regions are sharp and well defined. A UV excimer laser is capable of generating structure with well resolved features in the low micron range. Therefore, using this technique it is possible to generate biomolecule patterns on polymer surfaces with the same micron resolution.

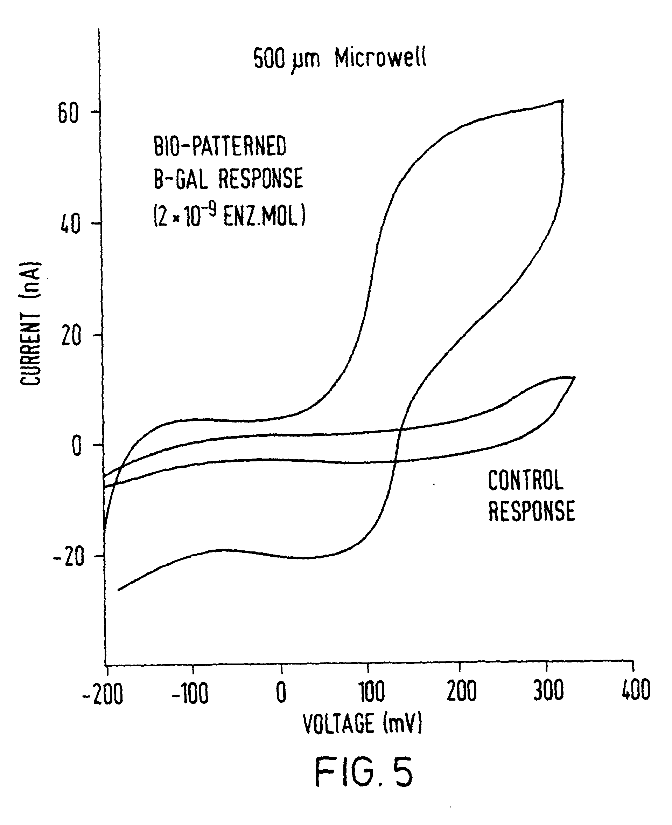

The enzyme β-Galactosidase may also be bio-patterned using the present invention and retains its enzymatic activity after immobilization onto UV-laser photoablated surfaces. β-Galactosidase was allowed to physically adsorb to photoablated polycarbonate through laser defined openings (1 mm diameter microwells) in a PET/PE protective laminate as previously described. After washing, the lamination was peeled off, leaving only laser-defined areas of immobilized β-Galactosidase. Substrate solutions (10 µL drops of aminophenylgalactoside (APG) in 0.2 M phosphate buffer, pH 7.4 with 1 mg / mL MgCl₂) were incubated for 2 hours at room temperature over photoablated microwells and control surfaces. A home built two electrode microprobe (working electrode = screen printed carbon, reference = screen printed Ag/AgCl paste) was then inserted into the substrate solutions and the potential of the working electrode was scanned at 50 mV sec⁻¹. The cyclic voltammogram for both the control (substrate over microwell with no β-Galactosidase) and test solution (substrate over microwell with β-Galactosidase) is shown in Figure 5. The conversion of APG to aminophenol and galactose can clearly be seen by the oxidative current at 200 mV indicating the presence of aminophenol and, therefore, β-Galactosidase activity. This experiment demonstrates that β-Galactosidase has been patterned within a photoablated microwell and that the immobilized species is still capable of enzymatic activity.

It should also be noted that the covalent attachment of affinity matrices may also be defined using UV-laser photoablation for purposes similar to those described above. Because the photoablative process is observed to produce chemically modified surfaces the photoablated, non-blocked section of polymer being defined is also available for covalent modification. Simple peptide linkages may be formed between these surface functional groups and amino acid side chains existing on protein structures such as enzymes and receptors. This may be accomplished by using commercially available reagents, such as Dicyclohexlcarbodiimide and N-Hydroxysuccinimide. Proteins, phospholipids, amino acids, and other components of interest may be attached in this manner or with other suitable methods of chemical attachment. It should be emphasized that if the initial blocking step is sufficient then any manner of these attachments will be localized to the region specified by the UV-laser machining process.

### Examples of Biomolecule Patterning by UV-laser Photoablation.

### Example 1

The UV-laser photoablation method described above can be used to pattern biological and other affinity matrices within microfabricated channels, networks of these channels, or on the surface of any suitable UV-absorbing substrate such as polymers, ceramics, etc. The present method is particularly advantageous for microfabricated channels in that it allows their precise delineation into reactive and non-reactive areas.

One embodiment of the present method involves the UV-laser fabrication of the desired microstructure, as described for example, in EP-A-0708331. This patterned surface is then protected or blocked, as depicted in Figure 2a, using a number of standard reagents which will depend upon the exact nature of the component to be deposited. As an example, a 5%, by weight, solution of NFDM is known to be sufficient for blocking a polystyrene surface, if an antibody is the reagent to be deposited and if the surface is to be used in an immunoassay. The thickness of the adsorbed blocking layer is approximately 10 nm, therefore, a very rapid laser machining process is all that is required to clean the surface in the area being defined. By using laser fluences of approximately 1.6 J cm⁻² per pulse, only a single pulse is required to effectively clean and eject the protected polymer substrate. In a preferred embodiment, 3 pulses are used to insure complete and consistent cleaning of the protein-coated substrate. This will leave a very thin (1 µm) cavity defining the region of interest, as is shown in Figure 2b. Subsequent reactions, by adsorptive deposition or covalent modification, will only occur in the UV-laser patterned region. As an example, the adsorption of antibodies 6 is depicted in Figure 2c in the laser defined region. Any antibodies adherent to the blocked polymer regions can simply be washed from the surface prior to sealing.

In an alternative embodiment of the invention, the initial unablated polymer substrate is completely blocked by first applying a laminate. In this embodiment, the laminate is a 35 µm PET/PE layer and is annealed to the base polymer at 125 °C for 2-3 seconds. The laser is used to etch a defined pattern into the laminate by firing repeated pulses sufficient to eventually pierce the laminate and create structures of desired depth into the base polymer. The resulting patterned laminate-substrate structures can then be easily immersed into solutions of affinity reagents. As an example, a protein receptor solution, composed of IgG antibodies dissolved in 200mM borate buffer (pH 9.0), may be incubated at room temperature for approximately two hours over the aforementioned structures. The structure is then washed with clean buffer, containing no antibodies, and the laminate is peeled off, as shown in Figure 3. All antibodies bound to the laminate are removed with the lamination, and the only remaining regions with adsorbed receptor are those defined by the UV-laser machining process. A small number of laser pulses can then be used on areas of the substrate where antibodies are not desired, thereby providing a cleaning step. This preferred method may, in some cases, be advantageous over the protein-blocking method, which is sufficient for many analytical applications but does not block 100 % of the reagent. In contrast, removal of reagents bound to the laminate by peeling leaves no possibility for adsorption in nonablated regions.

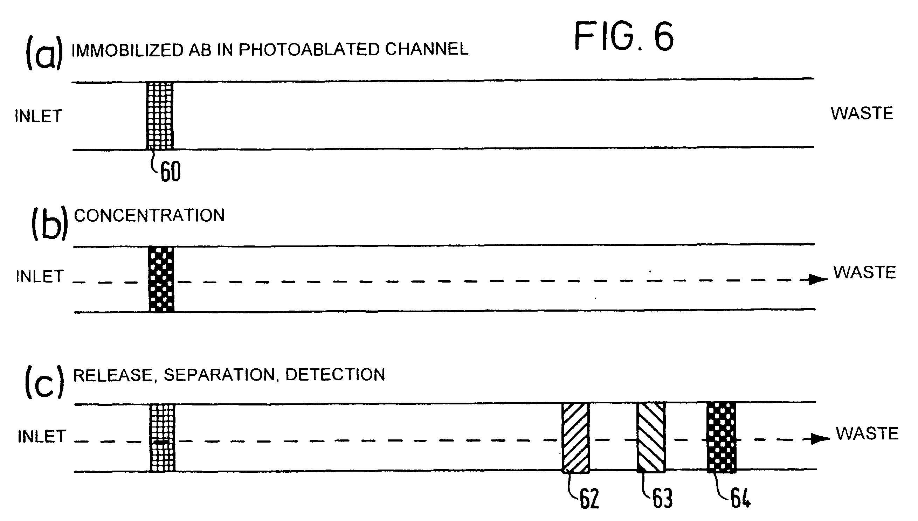

One possible embodiment of the invention utilizing a UV-laser defined reactive area within a microfabricated fluid handling system is shown in Figure 6. Here a biological affinity reagent 60, which may be any protein receptor (i.e., antibodies, enzymes, etc.), ligand, or nucleic acid (DNA, RNA, tRNA, etc.) can be defined in an area as small as 5 x 5 µm within a photoablated capillary 61. As an example, but by no means as a limiting case, an anti-polychlorinated biphenyl (anti-PCB) antibody may be deposited in such a zone. A sample containing several PCB congeners could then be introduced and continually pumped over the antibody zone, as shown in Figure 6b. Fluid pumping may be accomplished by any suitable means including electro-osmotic flow, positive pressure applied to the inlet, or negative pressure applied at the outlet.

This process serves two purposes. One, all molecules structurally similar to PCBs, sufficient to allow binding to the antibody receptor, are concentrated in this region over time as they are extracted from the flowing bulk solution. Two, the analytes of interests, PCB like molecules, are separated from complex background matrices which often interfere with their subsequent detection. After allowing sufficient time for concentration of the analyte, a release reagent such as octyl-glucopyranoside or any number of chaotropic agents can be injected onto the microchannel which would interfere with the antibody-ligand binding relationship. After release, capillary electrophoresis is carried out by applying high voltage across the microchannel, thereby separating only those congeners 62,63,64 (Figure 6c) which had an affinity for the antibody. This example is a possible application of laser-defined affinity regions within microchannels to extract, concentrate, separate, and then quantitate species of interest within a test solution. This pre-concentration step is used in this example to obtain a highly sensitive and well resolved electropherogram for quantitation of individual PCB congeners.

### Example 2

In another embodiment of the present invention a surface is patterned with nucleic acid probes, comprised of either deoxyribonucleic acids (DNA) or ribonucleic acids (RNA). The total array of the probes to be patterned by this invention will comprise numerous strands of genetic sequences encoding gene products of interest for the diagnosis of genetic disease, environmental exposure to toxins, retro-viral infection, etc.

For the present example, a preferred substrate will be polystyrene which is coated with a 35 µm thick PET/PE laminate (applied with pressure at 125 °C for 3 seconds). Polystyrene has been previously shown to physically adsorb DNA, and furthermore, in a fashion that allows subsequent hybridization¹¹. Photoablated polystyrene has now been shown to be advantageous for the immobilization of biomolecules and therefore provides a surface with greater immobilization capacity for nucleic acid probes. Therefore, adsorption will be used as a convenient immobilization procedure for nucleic acid probes used in this example. After lamination, small holes of approximately 10 µm in diameter are ablated through the protective layer with 200 pulses of 1.6 J cm⁻² per pulse. UV-laser photoablation is continued into the polystyrene substrate with a further 60 pulses creating 10 µm holes of approximately 10 µm in depth.

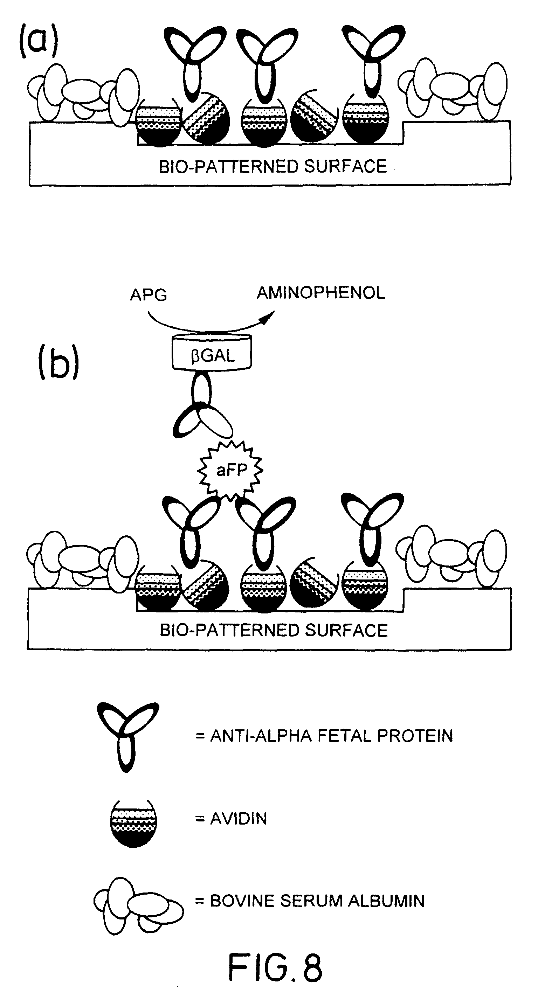

Solutions of nucleic acid sequences, prepared as known to those skilled in the art, which can be used as probes, are dissolved in high salt buffers (500 mM NaCl) and placed in small droplets (10 µL) over UV-laser ablated microwells for 2-4 hours at room temperature. After this initial incubation period the patterned microwell(s) is washed with buffer to remove any excess probe and then air dried. Using a step and repeat process different primary probe solutions are added to the growing array. One possible embodiment is shown in Figure 7, where a completed 2-dimensional array of microholes is shown with 100 µm spacing. Finally, the PET/PE protective laminate is peeled off removing any probe not immobilized in the microwell.

The device now consists of multiple photoablated wells 2-dimensionally defined with numerous different nucleic acid probes, capable of hybridization with sequences of medicinal and toxicological interest in a test sample. Subsequently, an unknown solution is placed in bulk over the entire array. Each of the immobilized probes will then specifically hybridize with a complementary sequence, if present, in the original test solution. A second set of probes, containing an easily visualized fluorescent marker and which will specifically bind to a different part of the test sequence, is then hybridized similarly. After binding the entire array may be stimulated with light of the proper wavelength in order to stimulate fluorescence, and the intensity of each well may be captured simultaneously using a CCD camera. Therefore, after analysis, the array will yield information as to the presence or absence of important genetic sequences in the test sample. The advantage of the present example is the ability to tightly pack and define multiple probes of interest, which in turn allows for the parallel testing of the same sample for many endpoints.

### Example 3

In another embodiment of the present invention a surface is patterned in a two step process, first with avidin and then with antibodies specific to an epitope of α-fetal protein (αFP).

For the present example, a preferred substrate will be polyimide which is coated with a 35 µm thick PET/PE laminate (applied with pressure at 125 °C for 3 seconds). Polyimide has been previously shown to physically adsorb proteins and as detailed above, has enhanced protein binding following UV-laser photoablation. Therefore, adsorption will be used as a convenient deposition procedure for immobilization of a monolayer of avidin receptor, which as previously described maintains its binding activity for its ligand, biotin, even after adsorption to UV-laser ablated polymer substrates.

The polyimide substrate is first cleaned with methanol and distilled water rinses followed by drying under pressurized air. The substrate is then laminated with a 35µm thick PET/PE laminate at 125 °C for 3 sec. After lamination, small holes of approximately 100 µm in diameter are ablated through the protective layer with 200 pulses of 2 J cm⁻² per pulse. UV-laser photoablation is continued into the polystyrene substrate with a further 200 pulses creating 100 µm holes of approximately 50 µm in depth. One possible embodiment is similar to that shown in Figure 7, but with a 10 x 10 microhole array, which has holes spaced 3 mm apart in either the vertical or horizontal dimensions. The larger dimensions described in the present example are for separation and easier addressability of the numerous test solutions to be applied. It should be noted though, that the entire "foot print" of such a test plate is only 3 x 3 cm whereas, a conventional 96-well microtiter plate is approximately 8.5 x 12.5 cm.

In a preferred sandwich immunoassay embodiment of the present example, a 500 µg / mL solution of avidin, from chicken egg whites, in PBS is allowed to incubate over the entire array for two hours at room temperature. The technique of pre-adsorbing avidin prior to application of a specific antibody solution allows the immobilization of any biotinylated antibody, receptor, or marker reagent. Furthermore, this technique will preserve more of the receptor's original activity by preventing close interaction with the substrate which leads to a certain amount of denaturation. If a biotinylated antibody reagent is unavailable or inconvenient to synthesize then the antibody can also be directly immobilized to the surface using the method of the present invention. In this case, after the avidin solution has adsorbed to the surface then the array plate is thoroughly washed with PBS and dried with pressurized air before application of the specific antibody solution.

In the present example, an antibody solution consists of anti-αFP in buffer ( 500 µg / mL in PBS), which is used for subsequent monitoring of neonatal health. It should be realized that this is a non-limiting case and that any suitable antibody can be substituted for anti-αFP, depending upon the desired analytical measurement. The solution of biotinylated anti-analyte antibody is allowed to incubate over the entire array for two hours at room temperature, thereby being strongly but non-covalently bound to the adsorbed avidin layer.

The anti-αFP -avidin coated microplate is cleaned by thorough rinsing with PBS and then the protective, patterned laminate is peeled off of the surface, leaving immobilized receptors only in the laser-patterned microholes. The entire surface (unablated polyimide and coated microwells) is then blocked with a solution of NFDM (10 mg/mL in 60 mM phosphate buffered saline, pH 7.0) at room temperature for 2 - 4 hours and then washed as previously described. At this stage the laser-patterned αFP detection plate is configured as shown in Figure 8a.

Various solutions of αFP can now be analyzed within the 10 x 10 microhole array. Various unknown serum solutions to be tested are diluted into PBS (between 1:1 and 1:10 depending on detection limit considerations) and 2 µL aliquots of these dilutions are placed over selected microwells in the array. These solutions are allowed to incubate for 30 minutes at room temperature. Because of the small diffusional environment existing in the microhole structures, the non-covalent binding reaction between the anti-αFP antibody and the αFP analyte will occur rapidly, relative to a reaction taking place in a conventional microtiter well. The plate is again washed with PBS as described earlier.

The final step in the assay of the present example is the addition of a second anti-αFP antibody that is specific for a different epitope than is the first antibody and which has been previously conjugated with an enzyme marker, such as β-Gal. Such enzyme-antibody markers are now often commercially available with specificity to various analytes of interest and if not, are readily prepared with heterobifunctional linkers capable of forming peptide bonds between the two proteins. A 2 µL aliquot of such an anti-αFP antibody-β-Gal conjugate is placed over various microwells which have been pre-incubated with unknown αFP test solutions. After incubation for 30 minutes at room temperature a second 2 µL aliquot of a β-Gal substrate solution (1 mM APG in 0.2 M phosphate buffer, pH 7.4 with 1 mg / mL MgCl₂) is allowed to react with the enzyme conjugate for a period of time that will be determined by the sensitivity desired of the assay. If the greatest sensitivity is desired than this time could be as long as 4 hours, however, if assay speed is the critical component than this time could be as short as 30 minutes.

A two electrode microprobe (working electrode = screen printed carbon, reference = screen printed Ag/AgCl paste), or any other suitable 2 electrode system capable of measuring 2-4 µL solutions, is then inserted into substrate solutions over the microwells. The potential of the working electrode is poised at +200 mV and the current is measured using a suitable commercially available current monitoring apparatus. The amount of current should be directly proportional to the amount of anti-αFP antibody-β-Gal conjugate that was bound to the microwells. The amount of conjugate is in turn directly proportional to the amount of αFP analyte that was bound to the UV-laser defined anti-αFP antibody primary coating. The final molecular configuration for detection of αFP is shown in Figure 8b.

Therefore, this example defines one possible use for the present invention of bio-patterning for the creation of microwell assay plates capable of detection of proteins of interest using the technique of sandwich immunoassay. The present method of patterning anti-analyte antibody is advantageous in that the diffusional environment for all reaction steps is reduced, the use of small volumes for samples and expensive reagents is facilitated, and the overall "foot print" of the assay plate is considerably reduced. The present method of patterning is also particularly advantageous in that there will be a large increase in the amount of primary antibody bound to the substrate relative to conventional polymer substrates. This will further increase the sensitivity of the assay as well as increase the reaction kinetics due to the large increase in the number of receptors.

### References

(1) Bhatia, S. K.; Teixeira, J. L.; Anderson, M.; Shriver-Lake, L. C.; Calvert, J. M.; Georger, J., H.; Hickman, J. J.; Dulcey, C. S.; Schoen, P. E.; Ligler, F. S. *Analytical Biochemistry* **1993,** *208,* 197-205.
(2) Arquint, P.; Koudelka-Hep, M.; van der Schoot, B.; van der Wal, P.; de Rooij, N. F. *Clinical Chemistry* **1994,** *40*, 1805-1809.
(3) Manz, A.; Graber, N.; Widmer, H. M. *Sensors and Actuators B* **1990,** 244.
(4) Koutny, L. B.; Schmalzing, D.; Taylor, T. A.; Fuchs, M. *Analytical Chemistry* **1996,** *68,* 18-22.
(5) Karube, I. , Universtiy of Twente, the Netherlands, 21-22 November 1994 1995; Kluwer Academic Publishers; 37-46.
(6) Manz, A. *Chimia* **1996,** *50,* 140-143.
(7) Walsh, M. K.; Swaisgood, H. E. *Journal of Food Biochemistry* **1993,** *17,* 283-292.
(8) Taylor, R. F. In *Protein Immobilization;* Taylor, R. F., Ed.; Marcel Dekker, Inc.: New York, 1991; Vol. 14, pp 377.
(9) Deshpande, S. S. In *Enzyme Immunoassays;* Chapman & Hall: New York, New York, 1996, pp 275-359.
(10) Brennan, M. In *Chemical & Engineering News,* 1996, pp 7.
(11) O'Donnell-Maloney, M.; Smith, C. L.; Cantor, C. R. *Trends in Biotechnology* **1996**, *14*, 401-407.
(12) Roberts, M. A.; Durst, R. A. *Analytical Chemistry* **1995,** *67,* 482-491.
(13) Roberts, M. A.; Bercier, P.; Girault, H. H. *Analytical Chemistry* **1996,** Submitted, September 1996.
(14) Smith, P. K. *Analytical Biochemistry* **1985,** *150,* 76-85.
(15) Siebert, T. A.; Reeves, S. G.; Durst, R. A. *Analytica Chimica Acta* **1993,** *282,* 297-305.
(16) Siebert, S. T. A.; Reeves, S. G.; Roberts, M. A.; Durst, R. A. *Analytica Chimica Acta* **1995,** *311,* 309-318.
(17) Plant, A. L.; Brizgys, M. V.; Locasio, B. L.; Durst, R. A. *Analytical Biochemistry* **1989**, *176*, 420-426.

## Claims

1. A method for immobilizing an affinity reagent onto UV-absorbing material **characterised by** using the process of UV-excimer laser induced photoablation, in combination with light patterning technology such as standard photomasks or fiber-optic light guides to create a rugged photoablated region of a UV-absorbing material facilitating binding of the reagent in a pattern corresponding to the region.

2. A method according to claim 1, wherein the affinity reagent comprises a biomolecule.

3. A method according to claim 1 or claim 2, wherein the laser photoablation creates, within the region, a chemically functionalized surface on the UV-absorbing material which shows enhanced immobilization capacity for biomolecules.

4. A method according to claim 3, wherein the UV-absorbing material is selected from polymers, including cellulose acetate, polystyrene, polycarbonate, poly(ethylene terephthalate), and polyimide.

5. A method according to claim 4, wherein the laser radiation, used for photoablation of polymers, is of a wavelength less than 330nm and energy fluences are greater than 0.5 J cm⁻² per pulse on the material surface.

6. A method according to any preceding claim, wherein the surface of the UV-absorbing material is masked by another material, capable of being photoablatively patterned by a UV-excimer laser.

7. A method according to claim 6, wherein said other material is selected from non-specific protein monolayers, chemically conjugated substances, or protective polymer laminates.

8. A method according to claim 6 or 7, wherein said other material forming a protective masking layer is patterned by UV-laser photoablation, creating one or more openings in a 2-dimensional pattern with µm (micron) resolution.

9. A method according to claim 8, wherein a solution of the affinity reagent is attached to the underlying UV-absorbing material via the UV-laser defined opening(s) through the masking layer.

10. A method according to claim 9, wherein said other material is a protective polymer laminate, which is removed after attachment of the affinity reagent to the UV-absorbing material.

11. A method according to claim 9 or 10, wherein the method of attachment is via physical adsorption, non-covalent binding, or covalent chemical conjugation.

12. A method according to claim 11, when dependent upon claim 2, wherein the biomolecule is a protein receptor, a carbohydrate conjugated protein, an enzyme, or a nucleic acid.

13. A method according to any preceding claim, wherein patterning occurs in 3 dimensions by using the UV-laser photoablative process to create structural depth upon which biomolecules may be subsequently be attached.

14. A biological or chemical sensor comprising a substrate of UV-absorbing material, carrying an affinity reagent in a specific pattern, **characterised in that** the reagent is attached to at least one rugged photoablated region of the substrate providing the pattern.

15. A sensor as claimed in claim 14, wherein the UV-absorbing material comprises a polymer.

16. A sensor as claimed in claim 15, wherein the polymer is selected from cellulose acetate, polystyrene, polycarbonate, poly(ethylene terephthalate) and polymide.

17. A sensor as claimed in claims 14, 15 or 16, wherein the affinity reagent comprises a biomolecule.

18. A sensor as claimed in claim 17, wherein the biomolecule is a protein receptor, a carbohydrate conjugated protein, an enzyme or a nucleic acid.

19. A sensor as claimed in any one of claims 14 to 18, wherein the or each said region comprises a microchannel.

20. A sensor as claimed in any one of claims 14 to 18, comprising an array of a plurality of said regions, each of which is a microwell.

## Patentansprüche

1. Verfahren zur Immobilisierung eines Affinitätsreagens auf UV-absorbierendem Material, **dadurch gekennzeichnet, daß** das Verfahren der UV-Excimerlaser-induzierten Photoablation in Kombination mit einer ein Lichtmuster erzeugenden Technik, wie z.B. Standard-Photomasken oder faseroptischen Lichtleitern, eingesetzt wird, um einen rauhen, photoablatierten Bereich aus einem UV-absorbierenden Material zu schaffen, wodurch die Bindung des Reagens in Form eines diesem Bereich entsprechenden Musters erleichtert wird.

2. Verfahren nach Anspruch 1, wobei das Affinitätsreagens ein Biomolekül umfaßt.

3. Verfahren nach Anspruch 1 oder Anspruch 2, wobei die Laserphotoablation innerhalb des Bereichs eine chemisch funktionalisierte Oberfläche auf dem UV-absorbierenden Material schafft, die eine verbesserte Immobilisierungskapazität zeigt.

4. Verfahren nach Anspruch 3, wobei das UV-absorbierende Material aus Polymeren, einschließlich Celluloseacetat, Polystyrol, Polycarbonat, Poly-(ethylenterephthalat) und Polyimid, ausgewählt ist.

5. Verfahren nach Anspruch 4, wobei die für die Photoablation von Polymeren verwendete Laserstrahlung eine Wellenlänge unter 330 nm aufweist und Energieflüsse von mehr als 0,5 J cm⁻² pro Puls auf der Materialoberfläche auftreten.

6. Verfahren nach einem der vorhergehenden Ansprüche, wobei die Oberfläche des UV-absorbierenden Materials von einem anderen Material, auf dem durch einen UV-Excimerlaser ein photoablatives Muster erzeugt werden kann, maskiert wird.

7. Verfahren nach Anspruch 6, wobei das andere Material aus nicht spezifischen Proteinmonolayern, chemisch konjugierten Substanzen oder Polymer-Schutzlaminaten ausgewählt ist.

8. Verfahren nach Anspruch 6 oder 7, wobei auf dem eine maskierende Schutzschicht bildenden anderen Material durch UV-Laserphotoablation ein Muster erzeugt wird, wodurch ein oder mehrere Öffnungen in einem 2-dimensionalen Muster mit einer Auflösung im µm (Mikron)-Bereich geschaffen werden.

9. Verfahren nach Anspruch 8, wobei eine Lösung des Affinitätsreagens auf das darunterliegende UV-absorbierende Material über die durch den UV-Laser definierten Öffnungen durch die maskierende Schicht hindurch aufgebracht wird.

10. Verfahren nach Anspruch 9, wobei es sich bei dem anderen Material um ein Polymer-Schutzlaminat handelt, das nach der Aufbringung des Affinitätsreagens auf das UV-absorbierende Material entfernt wird.

11. Verfahren nach Anspruch 9 oder 10, wobei das Aufbringungsverfahren über physikalische Adsorption, nichtkovalente Bindung oder kovalente chemische Konjugation erfolgt.

12. Verfahren nach Anspruch 11, in Abhängigkeit von Anspruch 2, wobei es sich bei dem Biomolekül um einen Proteinrezeptor, ein mit einem Kohlenhydrat konjugiertes Protein, ein Enzym oder eine Nukleinsäure handelt.

13. Verfahren nach einem der vorhergehenden Ansprüche, wobei ein Muster in drei Dimensionen unter Verwendung des UV-Laserphotoablationsverfahrens zur Schaffung von struktureller Tiefe gebildet wird, woraufhin Biomoleküle anschließend aufgebracht werden können.

14. Biologischer oder chemischer Sensor, der ein ein Affinitätsreagens in Form eines spezifischen Musters tragendes Substrat aus UV-absorbierendem Material umfaßt, **dadurch gekennzeichnet, daß** das Reagens auf wenigstens einen rauhen, photoablatierten Bereich des das Muster bereitstellenden Substrats aufgebracht ist.

15. Sensor nach Anspruch 14, wobei das UV-absorbierende Material ein Polymer umfaßt.

16. Sensor nach Anspruch 15, wobei das Polymer aus Celluloseacetat, Polystyrol, Polycarbonat, Poly-(ethylenterephthalat) und Polyimid ausgewählt ist.

17. Sensor nach Ansprüchen 14, 15 und 16, wobei das Affinitätsreagens ein Biomolekül umfaßt.

18. Sensor nach Anspruch 17, wobei es sich bei dem Biomolekül um einen Proteinrezeptor, ein mit einem Kohlenhydrat konjugiertes Protein, ein Enzym oder eine Nukleinsäure handelt.

19. Sensor nach einem der Ansprüche 14 bis 18, wobei der Bereich bzw. jeder der Bereiche einen Mikrokanal umfaßt.

20. Sensor nach einem der Ansprüche 14 bis 18, welcher ein Array aus mehreren der Bereiche umfaßt, wobei es sich bei jedem Bereich um ein Mikrowell handelt.

## Revendications

1. Procédé d'immobilisation d'un réactif d'affinité sur un matériau absorbant les UV, **caractérisé en ce qu'**on utilise le procédé de photoablation induite par laser UV à excimère, en combinaison avec une technologie d'application d'une configuration lumineuse telle que l'usage de photomasques standards ou de guides lumineux en fibre optique pour créer une région inégale, enlevée par photoablation, d'un matériau absorbant les UV afin de faciliter une liaison du réactif selon une configuration correspondant à la région.

2. Procédé selon la revendication 1, dans lequel le réactif d'affinité comprend une biomolécule.

3. Procédé selon la revendication 1 ou 2, dans lequel la photoablation au laser crée, dans la région, une surface chimiquement fonctionnalisée sur le matériau absorbant les UV, qui témoigne d'une capacité d'immobilisation renforcée pour les biomolécules.

4. Procédé selon la revendication 3, dans lequel le matériau absorbant les UV est choisi parmi les polymères, notamment l'acétate de cellulose, le polystyrène, un polycarbonate, le poly(téréphtalate d'éthylène) et un polyimide.

5. Procédé selon la revendication 4, dans lequel le rayonnement laser, utilisé pour la photoablation de polymères, a une longueur d'onde inférieure à 330 nm et les fluences d'énergie sont supérieures à 0,5 J cm⁻² par impulsion sur la surface du matériau.

6. Procédé selon l'une quelconque des revendications précédentes, dans lequel la surface du matériau absorbant les UV est masquée par un autre matériau capable d'être configuré par photoablation par un laser UV à excimère.

7. Procédé selon la revendication 6, dans lequel ledit autre matériau est choisi parmi des monocouches protéiques non spécifiques, des substances chimiquement conjuguées ou des laminés de polymères protecteurs.

8. Procédé selon la revendication 6 ou 7, dans lequel ledit autre matériau formant une couche de masquage protectrice est configuré par photoablation au laser UV, en créant une ou plusieurs ouvertures dans une configuration bidimensionnelle avec une résolution de l'ordre du micromètre (µm).

9. Procédé selon la revendication 8, dans lequel une solution du réactif d'affinité est fixée au matériau sous-jacent absorbant les UV via la ou les ouvertures définies par le laser UV à travers la couche de masquage.

10. Procédé selon la revendication 9, dans lequel ledit autre matériau est un laminé de polymère protecteur qui est retiré après fixation du réactif d'affinité au matériau absorbant les UV.

11. Procédé selon la revendication 9 ou 10, dans lequel le procédé de fixation se fait par adsorption physique, liaison non-covalente ou conjugaison chimique covalente.

12. Procédé selon la revendication 11, dans la mesure où elle dépend de la revendication 2, dans lequel la biomolécule est un récepteur de protéine, une protéine conjuguée à un hydrate de carbone, une enzyme ou un acide nucléique.

13. Procédé selon l'une quelconque des revendications précédentes, dans lequel la configuration se produit en trois dimensions en utilisant le procédé de photoablation au laser UV pour créer une profondeur structurelle sur laquelle des biomolécules peuvent être fixées ultérieurement.

14. Détecteur biologique ou chimique comprenant un substrat de matériau absorbant les UV portant un réactif d'affinité dans une configuration spécifique, **caractérisé en ce que** le réactif est fixé à au moins une région inégale du substrat qui a été soumise à une photoablation pour former la configuration.

15. Détecteur selon la revendication 14, dans lequel le matériau absorbant les UV comprend un polymère.

16. Détecteur selon la revendication 15, dans lequel le polymère est choisi parmi l'acétate de cellulose, le polystyrène, un polycarbonate, le poly(téréphtalate d'éthylène) et un polyimide.

17. Détecteur selon les revendications 14, 15 ou 16, dans lequel le réactif d'affinité comprend une biomolécule.

18. Détecteur selon la revendication 17, dans lequel la biomolécule est un récepteur de protéine, une protéine conjuguée à un hydrate de carbone, une enzyme ou un acide nucléique.

19. Détecteur selon l'une quelconque des revendications 14 à 18, dans lequel ladite ou chaque dite région comprend un microcanal.

20. Détecteur selon l'une quelconque des revendications 14 à 18, comprenant un réseau d'une pluralité desdites régions, dont chacune est un micro-puits.
